# EUROPEAN PATENT APPLICATION

(11) **EP 3 632 298 A1**
(43) Date of publication of application: **08.04.2020**
(21) Application number: 18198766.0
(22) Date of filing: 05.10.2018
(51) Int. Cl.: A61B 5/00, A61B 5/055, A63B 23/18

(54) **BREATHING ADAPTATION SYSTEM AND METHOD FOR INFLUENCING A BREATHING PARAMETER**

(71) Applicant: Koninklijke Philips N.V., 5656 AG Eindhoven (NL)
(72) Inventor: HOUBRAKEN, Mara, 5656 AE Eindhoven (NL); VAN EE, Raymond, 5656 AE Eindhoven (NL); LASCHET, Cliff Johannes Robert Hubertina, 5656 AE Eindhoven (NL); Timmers, Iris, 5656 AE Eindhoven (NL); COPPOLA, Giuseppe, 5656 AE Eindhoven (NL); BERGEVOET, Bas Arnold Jan, 5656 AE Eindhoven (NL)
(74) Representative: de Haan, Poul Erik

(57) **Abstract**

It is an object of the invention to improve a user's breathing for a specific application (e.g. diagnostic imaging, falling asleep improvement (shortening sleep onset), delivery, tinnitus control therapy, COPD, blood pressure control). This object is achieved by a breathing adaptation system configured for influencing a breathing parameter of a user's breathing pattern in order to meet a goal of decreasing or increasing the influenced breathing parameter to a certain extent in a user specific manner. The breathing adaptation system comprises a sensor, configured for monitoring a current value of the influenced breathing parameter of the user. The breathing adaptation system further comprises a feedback unit configured for providing feedback to the user about a preferred value of the influenced breathing parameter, wherein the preferred value is different from the current value of the influenced breathing parameter (310). The breathing adaptation system further comprises a control unit, comprising program code means for determining the preferred value of the influenced breathing parameter in a user specific manner by means of an intelligent agent, wherein the control unit is implemented such that the intelligent agent is rewarded (330) by means of a reward function after performing the determination of the preferred value (320) of the influenced breathing parameter, wherein the reward function is such that it balances the reward for obtaining the goal and the ability of the user to breathe according to a breathing pattern having the preferred value for the influenced breathing parameter.

## Description

### FIELD OF THE INVENTION

The invention relates to influencing of a user's breathing parameter and more specifically to influencing the breathing parameter of a user for the purpose of diagnostic imaging and therapeutic treatment.

### BACKGROUND OF THE INVENTION

A user's (e.g. a patient's) breathing pattern may have a large effect on the quality of medical images acquired or a therapeutic treatment delivered to the user or patient. This effect is due to the motion of the organ and that medical image registration may benefit from stable or predictable motion. Some imaging or therapy protocols may rely on breath hold or breath gating. Longer breath hold periods may be beneficial, because this allows for a longer data acquisition. Further, imaging and / or therapy delivery may be improved when breathing frequency and / or amplitude are stable and / or predictable. Examples of such therapies are radiation or proton treatment.

In order to improve image quality US2013/0211236A1 describes a system for MRI data acquisition using physiological monitoring. The system described can be used to provide instructions to the patient. These instructions could for example be "stop breathing" or "please hold" and / or hyperventilation commands.

Breathing is probably the only part of the autonomous nervous system that we can actively influence in our response to stress - unlike heart rate or skin conductance. Therefore, besides diagnostic image quality specific breathing patterns may also affect other situations, for example a time needed to fall asleep (shortening sleep onset), tinnitus control therapy, pain experienced by pregnant women during delivery, blood pressure control, and /or [COPD].

### SUMMARY OF THE INVENTION

It is an object of the invention to improve a user's breathing for a specific application (e.g. diagnostic imaging, falling asleep improvement (shortening sleep onset), delivery, tinnitus control therapy, COPD, blood pressure control).

At present breathing can be dealt with during diagnostic imaging and / or therapy delivery in several different ways.

First of all, one could provide all patients with the same instructions with respect to breathing pattern and / or breath hold. The disadvantage of this approach is that there is a large variation between different patients, which means that for some patients it may be very difficult to follow the instructions resulting in suboptimal image quality or therapy delivery. Other patients may be better capable of maintaining breath hold, but by providing instructions that are based on an average or poor performing patient, these capacities of a patient are not used, resulting in a reduced image quality for this specific patient compared to what could have been possible or resulting in a longer scan time than necessary.

Alternatively, a free breathing protocol may be used. A free breathing protocol requires a certain level of predictability of the breathing pattern. A diagnostic setting may be stressful for the patient. As a result the breathing pattern may become (very) irregular, which makes it hard to predict. Therefore, a free breathing protocol does not work optimal for all patients.

When using the free breathing protocol, imaging may be adapted to the patient's (current) breathing pattern, as described in US2013/0211236A1. This option has the advantage that it may improve image quality for the specific patient and it may in addition improve patient comfort.

It is an insight of the inventors that image quality may be further improved by a breathing adaptation system according to claim 1, a method according to claim 14 or a computer program product according to claim 15. All embodiments described herein in relation to claim 1 are also relevant for the method according to claim 14 and the computer program product according to claim 15. The method according to claim 14 and computer program product according to claim 15 can be adapted to incorporate the embodiments described herein.

The breathing adaptation system according to claim 1 is further configured to be used to guide a user to a user-specific breathing pattern that is improved or optimal for a specific application, like e.g. sleep improvement, delivery, tinnitus therapy, blood pressure control, and COPD. It is an insight of the inventors that breathing patterns may be optimized for the individual user or patient by the use of intelligent agents. The intelligent agent can be used to optimize one or more breathing parameters. These parameters will herein also be called influenced breathing parameters. Breathing parameters that are not optimized by the agent may herein be called non-influenced breathing parameters. Examples of breathing parameter are, frequency, phase, amplitude, duration of breath hold, duration of inhale phase, duration of exhale phase, repeatability of breathing pattern etc. For example, the breathing amplitude maybe increased or decreased. A decreased amplitude maybe beneficial for diagnostic imaging because it results in reduced motion and / or therapy delivery. Less movement may have a positive impact on the predictability of the movement to an exact location. Also a decreased amplitude may be beneficial for e.g. tinnitus and blood pressure. Further, the breathing frequency may be increased or decreased. A decreased breathing frequency may help during breath gating or may make a user more relaxed, which may help to fall asleep or experience less pain, and / or reduce blood pressure. Further, the agent may especially stimulate an increase or decrease of the inhale or exhale phase. An increased exhale phase may be beneficial for medical image acquisition. Also, the repeatability of the above mentioned parameters may be increased, which could be beneficial for diagnostic imaging and / or therapy delivery. Further, the breathing parameter could also be the duration of a breath hold. An increased duration of a breath hold can be advantageous for imaging and therapy. However, as explained below, it could also be relevant to know what is the maximum (comfortable) duration of a breath hold of a patient. Decreasing of increasing any of the above mentioned parameters to a certain extent for the individual user could be a goal of the breathing adaptation system. To a certain extent could for example be to a certain absolute value or to a certain value relative to the initial value of the influenced breathing parameter, also it could be substantially close to the maximum or minimum parameter value achievable by the individual user or patient. The intelligent agent will try to obtain the goal by providing guidance to the patient with respect to a preferred adaptation of his breathing pattern. After an action of the intelligent agent, breathing parameters of interest will be measured. Based on these measured parameters the intelligent agent will be positively or negatively rewarded. The reward will be calculated by means of a reward function. The reward will be higher if the breathing pattern has changed such that the breathing pattern is now closer to the goal. When the influenced breathing parameter has changed in the wrong direction, the reward will be lower or even negative.

It is a further insight of the inventors that good results are achieved when choosing a reward function that balances the reward for obtaining the goal and the ability of the user to breathe according to a breathing pattern having the preferred value for the influenced breathing parameter. The ability of the user to breathe according to a breathing pattern having the preferred value for the influenced breathing parameter is reflected by the user's ability to follow the breathing pattern having said breathing parameter determined by the intelligent agent. By taking the ability to follow into account, user comfort is increased. When applying this, it appears that some users can go to a controlled breathing rhythm very fast, whereas others should be gently guided into it. It is more motivating for the user when he has the feeling that he can follow the rhythm. This may in turn lead to improved results, e.g. in terms of image quality, sleep onset, experienced pain etc.

Preferably, the feedback is provided to the user in a multisensory fashion, for example audio-visual or audio-tactile. This is advantageous, because the inventors have realized that it is important that the "feed-back" information in a biofeedback system according to the invention is fully attended by the user. The biofeedback method would suffer from distracted attention/focus of the user, for example from an MRI scanner during diagnostic imaging. In order to increase focus of the patient to the fed-back breathing information the feedback-information is preferably a rhythmically synchronous combination of at least 2 perceptual modalities simultaneously.

The reason for this is that heteromodal congruency is an efficient means for the brain to identify signal relevance in the bombardment of sensory signals. There is a machinery of neurons especially devoted to combine perceptual functions across sensory modalities. These neurons play an important role in perceptual control to influence the conscious experiences. Signal congruency, then, may facilitate multimodal mechanisms of voluntary perceptual control, since there is more support for a particular percept when there is information from another sensory modality that is congruent with it. Experiments revealed that the capacity to voluntarily select one of two competing percepts is greatly enhanced when there is such congruency (in some observers over 400% increase in control over perception). This latter finding indicates that a feedback system based on multimodal congruency may be able to voluntarily control perceptual focus of experience away from other distractions, such as pain, anxiety, scanner noise etc.

Visual feedback maybe provided e.g. by displaying a breathing curve having one or more preferred parameters. Also, light intensity and / or color may be changed in such a way that it reflects the one or more breathing parameters. According to further embodiments a shape may change such that it reflects the one or more breathing parameters. For example the amount of change and the frequency of change could depend on the one or more breathing parameters. According to embodiments of the invention the shape used are shapes that are perceived to have a relaxing effect, for example the shapes could relate to nature, for example like waves of the sea.

The auditory feedback could for example be in the form of verbal instructions (e.g. breathe in / breathe out), but could also be just a sound (pip) to mark a certain position in the breathing cycle. Also for example, it could be a melody having a certain frequency related to the current or preferred frequency. Also, the pitch could be changed in order to reflect the one or more breathing parameters. According to embodiments, the sounds are chosen such that they relate to the visual feedback, for example the sound of waves (e.g. created by means of pink noise) could be provided to the user in combination with a display of waves. Another example is the use of randomly generated noise for this purpose. This is advantageous, because as explained above the user maybe more likely to follow the multisensory feedback when it is in agreement with each other.

Tactile feedback could be provided for example by placing one or more actuators in a matras, pillow or other device that mimic the motion of breathing.

Preferably, the different sensory feedbacks will have the same amplitude and / or frequency. Preferable, the feedback signals will always have a frequency at or below ∼1 Hz. Such low frequency (multi-sensory) stimulations cannot be produced 'within one's body', therefore it is favorable for a patient to stay attentive to such a stimulation.

According to embodiments of the invention the feedback unit is configured for providing a current value of the influenced breathing parameter to the user for a period of time before starting to provide the preferred value of the influenced breathing parameter. By providing the user with his current breathing pattern before starting to influence it, may give the user an improved feeling of control, which in turn may result in a larger influence of the breathing parameter in the end.

According to further embodiments of the invention the breathing adaptation system is configured for repeating the determination of the preferred value of the influenced breathing parameter and providing this to the user until a predefined criterion is met. Such criterion could for example be a 10% increase or decrease of a breathing parameter like e.g. the breathing period. The criterion could also be: a certain breath hold duration, or the longest breath hold the patient is still comfortable with.

The advantage of the breathing adaptation system, in particular for diagnostic imaging and / or treatment delivery, may not only be in the predictability of the user's or patient's breathing, the breathing adaptation system may in addition have the advantage that the patient or user becomes more relaxed, because he can focus on the breathing stimulation instead of on stress. In diagnostic imaging or therapy delivery this may increase the likelihood of a patient being able to finish the scan or treatment and / or may reduce the risk of sudden movements affecting image quality. Also, in particular in younger patients, it may reduce the need for sedation. Nowadays, as described in several reports 1 in 8 or 1 in 10 patients is sedated before undergoing an MRI exam.

The above mentioned advantage of a more relaxed patient may be even increased when the patient is better prepared to the situation he is going to be exposed to e.g. during image acquisition and / or treatment delivery. This can be achieved by providing the patient with a breathing adaptation system according to claim 1. Also the patient can be provided with a computer program product according to claim 15, which can be run on for example on a computer, tablet or a smartphone. A separate or built in camera could be used as the sensor needed for monitoring the influenced breathing parameter. By such better preparation of the patient, the patient may become more relaxed and may therefore achieve better results in the actual clinical setting (e.g. delivery, diagnostic imaging, therapy delivery). This may in turn reduce total scan time and the need of anesthesia or sedation, which is particularly relevant for children. An addition or alternatively, the breathing adaptation system may be offered in the waiting room for the diagnostic imaging or therapy delivery, potentially resulting also in a more relaxed and well-prepared patient.

According to further embodiments, the breathing adaptation system is configured for sharing a final preferred value of the influenced breathing parameter with a diagnostic imaging center. This embodiment is advantageous, because it may help the diagnostic imaging center (e.g. a hospital) in scheduling a time-slot for image acquisition and / or therapy delivery. For example more time may be needed for patients that are not capable of breathing in a regular fashion and / or who are not capable of maintaining a long breath hold. Also this information may be used to optimize the imaging protocol to the individual patient's breathing pattern or breath hold. This may be achieved for example as proposed in US2013/0211236A1. For example, in case it is expected that the patient can only hold his breath for 7 seconds, wherein an actually selected imaging protocol would require a breath hold of at least 15 seconds, this actually selected imaging protocol may be exchanged by a new imaging protocol which requires a data acquisition time of 7 seconds. The new imaging protocol may acquire the essential information in the first 7 seconds with the possibility to extend acquisition time in case that the patient can hold his breath longer successively improving image quality. The image protocol may be adapted to the patient in other ways as well, for example the signal-to-noise ratio, the contrast, SENSE factor, k-space trajectory, epi factor etcetera may be adapted such that they are optimized to the patient specific breathing parameters.

Also, based on the shared final preferred value it can be decided if a breath hold regime is the best option for the patient, or whether gated, free and / or controlled breathing imaging and / or therapy delivery are a better option for this specific patient. Based on the shared final preferred value for different regimes an estimate can be made about the achievable image quality and scan time for the different regimes.

According to embodiments of the invention, the breathing adaptation system further comprises a patient scheduling module configured for scheduling a timeslot for diagnostic imaging, wherein the length of the timeslot is dependent on the final preferred value of the influenced breathing parameter.

According to further embodiments of the invention, the breathing adaptation system further comprises an image protocol optimizer, wherein the image protocol optimizer is configured to optimize one imaging parameter at least partly based on the final preferred value of the influenced breathing parameter.

These and other aspects of the invention will be apparent from and elucidated with reference to the embodiments described hereinafter.

### BRIEF DESCRIPTION OF THE FIGURES

Figure 1 diagrammatically shows an intelligent agent placed in a certain environment and
Figure 2 diagrammatically shows a breathing adaptation system according to embodiments of the invention and
Figure 3 displays the breathing frequency for one person over time, as well as the preferred value of the breathing frequency determined by the control unit and the corresponding reward figure and
Figure 4 diagrammatically shows a breathing adaptation system for diagnostic imaging according to embodiments of the invention and
Figure 5 diagrammatically shows a method for influencing a breathing parameter according to the invention.

### DETAILED DESCRIPTION OF THE INVENTION

Reinforcement learning is a field in machine learning that is inspired by behaviorist psychology, and is concerned with how an intelligent agent can take actions in an environment so as to maximize its cumulative reward.

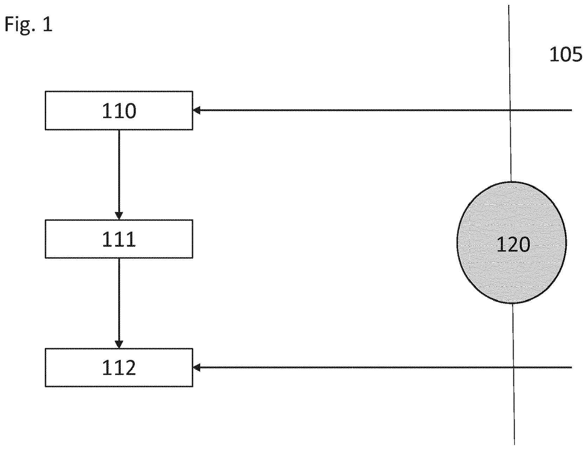

Figure 1 diagrammatically shows an intelligent agent 111 placed in a certain environment 105. It can perceive the environment through sensors 110 to observe the current state of the environment. The agent can also act upon the environment using its actuators 112, and observes a certain reward 120, that is determined by the previous action and how good or bad the current state of the environment is. A good state yields a positive reward, whereas a bad state will yield a less positive or even negative reward. The reward is used to describe an implicit goal. Using the rewards, the intelligent agent 111 can determine what actions are preferred to achieve the goal. Reinforcement learning can be seen as the reasoning of the intelligent agent: it determines what action to take in a certain state. The intelligent agent does this by estimating the value of an action in this state, and by exploring states and actions for some iterations. This estimate becomes more accurate and an optimal policy can be defined: the action that maximizes the value of the next state will be picked.

To reach an optimal policy, the intelligent agent will often first go through a stage called exploration, where it will explore different actions regardless of the estimate of the value for each action. However, once this estimate has reached an acceptable accuracy, the agent should switch to a stage called exploitation. In this stage, it will only select the action with the highest value. In reality, there is no such clear boundary between the exploration and exploitation stages. It is more likely that the agent will start with a fully explorative policy and slowly move towards a more exploitative policy. However, preferably the agent should always keep some explorative actions in its policy.

Usually, the agent initiates the estimates of the values randomly. In order to improve on that, domain knowledge about the environment can be added to the reasoning to form an initial policy. This ensures that the agent will take less time to converge to an optimal policy.

There are several reasoning algorithms available in the prior art among one is Q-learning, which utilizes the Bellman equation to estimate the value of executing a certain action in a certain state (a state-action pair). This algorithm assigns a Q-value to each state-action pair by taking the reward obtained in the state into account, plus the expected Q-value of the best action in the next state, multiplied by a decay factor. This decay factor is a numeric value between 0 and 1. The expected Q-value of the next state is multiplied by the decay factor since the agent has to take one more action at a certain cost to reach this Q-value.

The best action in a certain state is computed by calculating the Q-values for all the possible actions, and choosing the action with the highest Q-value. The optimal policy corresponds to a policy where the expected value of the total reward return over all successive steps, starting from the current state, is the maximum achievable.

An alternative method to Q-learning is to search directly in policy space, instead of estimating a value for each possible action. Policy gradient is such a known method, based on optimizing parametrized policies by gradient descent.

In order to estimate an approximately correct value of a certain state, approximators can be used. These could for example be decision trees or random forest, both are known in the art.

When using an intelligent agent for influencing breathing parameters it is preferable to smooth the data that is sensed by the sensor in such a way that relevant patterns are captured while white noise is filtered out. Smoothing methods are known in the art among which are rectangular and triangular smoothing. It is further preferable to normalize the data sensed by the sensor.

Preferably, the data sensed by the sensor is modeled in order to extract the important breathing parameters. However, depending on the type of sensor and the goal, the one or more breathing parameters may also be measured directly. The breathing pattern could for example be modeled by sine or cosine function having parameters describing the amplitude, period and / or phase shift of the breathing pattern sensed by the sensor.

The current state of the breathing parameter(s) may be monitored by regular intervals. These intervals should not be too short, such that the user or patient has sufficient time to act upon the determined preferred value of the influenced breathing parameter. However, preferably this interval is neither too long, because this would make the method take too long.

The actions that the intelligent agent can take are preferably defined by the same parameters as a state is defined, which are for example amplitude, period and / or phase. Preferably, the actions that the intelligent agent is allowed to perform are dependent of the current state. In order to prevent too strong hyperventilation or too slow breathing, the value for the breathing frequency or interval may be constrained.

The reward function used is such that the reward balances the reward for obtaining the goal and the ability of the user to breathe according to a breathing pattern having the preferred value for the influenced breathing parameter. For example, the goal could be reducing the breathing parameter breathing frequency. In this example, the reward function should be designed such that it rewards lower breathing frequencies more than higher breathing frequencies. Also, alternatively or additionally the reward function in this example may be designed such that it rewards a decrease in breathing frequency compared to a previous state.

The ability of the user to breathe according to the breathing pattern having the preferred value for the influenced breathing parameter has to be taken into account in the reward function as well. This could for example be achieved by taking into account the difference between the preferred value of the influenced breathing parameter and the actually measured or sensed value for this influenced breathing parameter.

Alternatively, the ability of the user to breathe according to the breathing pattern could be determined in a broader sense. For example the breathing adaptation system could calculate an "ideal" breathing pattern using the measured values for the user's breathing frequency, amplitude and / or phase shifts. This could for example be achieved by using the above mentioned (co)sine function.

When calculating the ideal breathing pattern, preferably constraints could be provided to the non-influenced breathing parameters, such that they remain in a range that is assumed to be comfortable for the patient. When the measured non-influenced breathing parameters are within the allowed range, the breathing adaptation system will use the user's values when calculating the ideal breathing pattern. However, if the measured values are above or below the values set by the constraints, values taken from the allowed range will be used for calculating the ideal breathing pattern. Usually these values will be the upper or lower limit of the allowed range.

The control unit could then determine the difference between the calculated ideal breathing pattern and the actual breathing pattern of the patient / user. The difference could for example be expressed in a sum of squared differences, but alternatives are possible. In this way not only the breathing parameter that is being optimized (e.g. frequency) is taken into account, but also other parameters that may have an effect on the patient's comfort (e.g. breathing amplitude) when assessing the patient's comfort. Also, other irregularities in the measured breathing parameter could be detected in this way and be fed back to the intelligent agent by means of the reward function.

In order to obtain a value for the measured breathing parameter(s) it is advantageous to take into account a (weighted) average over multiple breathing cycles of the user or patient.

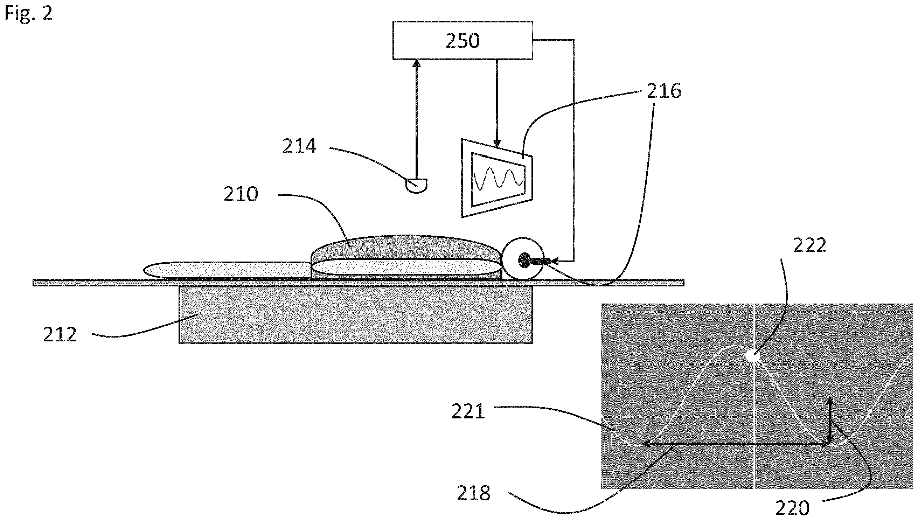

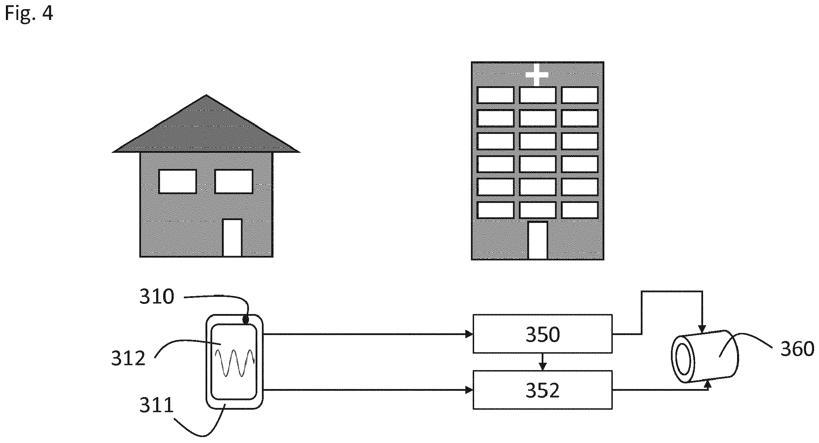

Figure 2 diagrammatically shows a breathing adaptation system according to embodiments of the invention. A patient / user 210 is positioned on a bed 212. The bed could for example be the bed of a medical imaging system. The user is breathing according to a certain breathing pattern that is sensed by a sensor 214, which in this case is a camera. In this case this camera is displayed as a separate camera, which could for example be a camera inside a bore of a medical imaging system. However, in other applications it could also be for example the camera figure 4, 310 of a smartphone or tablet. The sensor does not need to be a camera. It could also be any other sensor suitable for determining breathing parameters, like for example a breathing bellow. The value for the breathing parameter may be directly measured by the sensor. Otherwise a mathematical function, e.g. a sine or cosine, may be fitted to the breathing pattern measured by the sensor. This measured breathing pattern is preferably smoothed and normalized. The data sensed by the sensor are sent to the control unit 250, which comprises the intelligent agent figure 1, 111. The control unit is connected to a feedback unit 216, configured for providing feedback to the user 210. In this embodiment the feedback is audiovisual. The visual feedback in this embodiment is provided by displaying a breathing pattern. This feedback reflects one or more of the breathing parameters, like for example the breathing period and / or the breathing amplitude 220. The line and circle 222 in this embodiment indicate a current position in the breathing cycle of the user. The part of the breathing pattern on the right side of this line and circle 222 display a breathing pattern according to the preferred value of the influenced breathing parameter. So, for example in case the influenced breathing parameter is breathing amplitude, the right hand side of the circle and line 222 displays a breathing pattern having a breathing amplitude according to the preferred value of the breathing period. According to embodiments of the invention, the breathing adaptation system could be configured such that at the beginning of the method not the preferred value of the influenced breathing parameter is displayed, but only a current value of the influenced breathing parameter. This would mean that the actual value of the breathing parameter of the user breathing cycle would be provided to the user. This would mean that the user would always breathe according to the breathing parameter displayed during this phase. This may give the user an increased feeling of being in control.

When the preferred value of the influenced breathing parameter is provided to the user, the sensor keeps on measuring the values of the influenced breathing parameter achieved by the user. Depending on how well the user is capable in adapting to the preferred value of the influenced breathing parameter, the control unit 250 will not change the value for the preferred value of the influenced breathing parameter at all or will increase or decrease it in a faster or slower fashion.

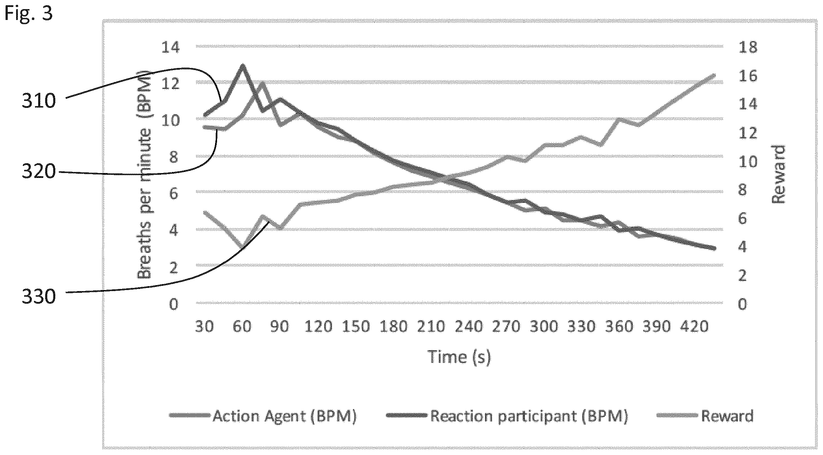

Figure 3 displays the measured breathing frequency 310 for one person over time, as well as the preferred value of the breathing frequency 320 determined by the control unit figure 2, 250 and the corresponding reward figure 330. The measured breathing frequency starts with around 10 breaths per minute. The control unit has as a result determined that the preferred value of the influenced breathing parameter should be around 9.5 breaths per minute. The user is however not capable of adapting to this preferred breathing frequency and starts breathing with more than 12 breaths per minute. The control unit adapts accordingly and determines a preferred value for the breathing frequency of 12 breaths per minute. After this the user appears better capable to pick up the preferred breathing frequency and after one more slight increase of the measured breathing frequency, the measured breathing frequency goes more or less steadily downwards until a breathing frequency of about 3 breaths per minute is reached. This improvement of the influenced breathing parameter is also reflected in the reward 330, which increases over time.

Figure 4 diagrammatically shows a breathing adaptation system configured to be used for the purpose of improving diagnostic imaging or treatment delivery. A patient scheduled for diagnostic imaging will be provided with a computer program product, e.g. an app. The computer program product can be used at home for example on a computer, tablet or phone 311. The patient may be provided with a sensor that is connectable to the computer program product and is configured to measure the patient's breathing parameters. Alternatively, a built-in camera 310 of the phone or tablet may be used as sensor to measure the breathing parameters. The device used by the patient 311 may display breathing parameters to the patient, e.g. in the form of a breathing pattern 312. Similarly, as described above based on measured breathing parameter values, the control unit will start to influence the patient's breathing pattern. This may be a good way for the patient to prepare for the upcoming diagnostic exam. Also this preparation may provide the patient and the caregiver with the confidence that the patient will be capable of finishing the imaging exam or therapeutic session without the need for sedation. After a certain breathing rate criterion is met, the control unit stops influencing the patient's breathing pattern and shares a final value of breathing parameters of interest to an image protocol optimizer 350 and / or a patient scheduling module 352 that may be located at the hospital. As described above, based on the shared final value of the breathing parameters the image protocol optimizer may be configured for optimizing the image protocol to the patient specific breathing parameters. This optimization may be done manually or (semi)-automatically. This image protocol may in turn be shared with the patient scheduling module which uses this information for scheduling a timeslot for imaging this patient. For example the patient scheduling module may determine an expected length of the timeslot for this specific patient, also it may determine whether specific (more qualified) personnel may be needed during scanning or preparation of this particular patient or whether access to sedation or anesthesia may be needed. The patient scheduling module does not need to be connected to the imaging protocol optimizer, but may also schedule based on the output of the computer program product alone, e.g. based on these values the patient scheduling module may be able to assess whether additional preparation time may be needed.

The diagnostic imaging system (e.g. MRI system) 360 will receive input from the image protocol optimizer. The diagnostic imaging system does also comprise the breathing adaptation system according to claim 1 and will hence be configured for influencing the patient's breathing pattern.

Of course, the breathing pattern at home may not be the same as the breathing pattern during actual diagnostic imaging, but the output from the computer program product may give an indication of the patient's breathing pattern during diagnostic imaging or treatment delivery.

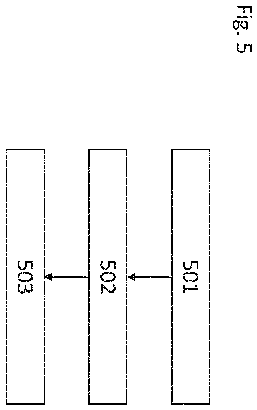

Figure 5 diagrammatically shows a method for influencing a breathing parameter according to the invention. The method comprises the steps of:
- monitoring a current value of the influenced breathing parameter of the user 501 and;
- providing feedback to the user about a preferred value of the influenced breathing parameter, wherein the preferred value is different from the current value of the influenced breathing parameter 502 and;
- determining the preferred value of the influenced breathing parameter in a user specific manner by means of an intelligent agent, wherein the intelligent agent is rewarded by means of a reward function after performing the determination of the preferred value of the influenced breathing parameter, wherein the reward function is such that it balances the reward for obtaining the goal and the ability of the user to breathe according to a breathing pattern having the preferred value for the influenced breathing parameter 502.

Whilst the invention has been illustrated and described in detail in the drawings and foregoing description, such illustrations and description are to be considered illustrative or exemplary and not restrictive; the invention is not limited to the disclosed embodiments.

## Claims

1. A breathing adaptation system configured for influencing a breathing parameter of a user's breathing pattern in order to meet a goal of decreasing or increasing the influenced breathing parameter to a certain extent in a user specific manner, wherein the breathing adaptation system comprises
- a sensor, configured for monitoring a current value of the influenced breathing parameter of the user and;
- a feedback unit configured for providing feedback to the user about a preferred value of the influenced breathing parameter, wherein the preferred value is different from the current value of the influenced breathing parameter and;
- a control unit, comprising program code means for determining the preferred value of the influenced breathing parameter in a user specific manner by means of an intelligent agent, wherein the control unit is implemented such that the intelligent agent is rewarded by means of a reward function after performing the determination of the preferred value of the influenced breathing parameter, wherein the reward function is such that it balances the reward for obtaining the goal and the ability of the user to breathe according to a breathing pattern having the preferred value for the influenced breathing parameter.

2. A breathing adaptation system according to claim 1, configured for providing the feedback in the form of a multi-sensory feedback, wherein the frequencies of different sensory feedbacks are equal.

3. A breathing adaptation system according to any of the preceding claims, wherein the feedback unit is configured for providing a current value of the influenced breathing parameter to the user for a period of time before starting to provide the preferred value of the influenced breathing parameter.

4. A breathing adaptation system according to any of the preceding claims, wherein the feedback unit is configured for providing a current or preferred value of the influenced breathing parameter to the user by providing a breathing pattern, which is based on the current or preferred value of the influenced breathing parameter.

5. A breathing adaptation system according to any of the preceding claims, configured for repeating the determination of the preferred value of the influenced breathing parameter and providing this to the user until a predefined criterion is met.

6. A breathing adaptation system according to any of the preceding claims, wherein the feedback signal provided has a frequency below 1 Hz.

7. A breathing adaptation system according to any of the preceding claims, wherein the influenced breathing parameter is reflecting breathing amplitude, breathing frequency and / or repeatability of breathing amplitude and / or breathing frequency, and / or breathe hold duration.

8. A breathing adaptation system according to any of the preceding claims, configured to be used for the purpose of improving diagnostic imaging or treatment delivery.

9. A breathing adaptation system according to claim 8, further comprising a diagnostic imaging system and / or a treatment delivery system.

10. A breathing adaptation system according to claim 9, wherein the diagnostic imaging system is an MRI system.

11. A breathing adaptation system according to claims 5 and 8, configured for sharing a final preferred value of the preferred influenced breathing parameter with a diagnostic imaging or treatment center.

12. A breathing adaptation system according to claim 11, further comprising a patient scheduling module configured for scheduling a timeslot for diagnostic imaging, wherein the length of the scheduled timeslot is dependent on the final preferred value of the influenced breathing parameter.

13. A breathing adaptation system according to claim 11 or 12, further comprising an image protocol optimizer, wherein the image protocol optimizer is configured for optimizing one imaging parameter at least partly based on the final preferred value of the influenced breathing parameter.

14. A method for influencing a breathing parameter of a user's breathing pattern in order to meet a goal of decreasing or increasing the influenced breathing parameter to a certain extent in a user specific manner, wherein the method comprises the steps of
- monitoring a current value of the influenced breathing parameter of the user and;
- providing feedback to the user about a preferred value of the influenced breathing parameter, wherein the preferred value is different from the current value of the influenced breathing parameter and;
- determining the preferred value of the influenced breathing parameter in a user specific manner by means of an intelligent agent, wherein the intelligent agent is rewarded by means of a reward function after performing the determination of the preferred value of the influenced breathing parameter, wherein the reward function is such that it balances the reward for obtaining the goal and the ability of the user to breathe according to a breathing pattern having the preferred value for the influenced breathing parameter.

15. A computer program product comprising program code means for performing the method according to claim 14.
